Europäisches Patentamt

(19) European Patent Office

Office européen des brevets

(11) Publication number: **0 214 865 B1**

# EUROPEAN PATENT SPECIFICATION

(12)

(45) Date of publication of patent specification: **23.01.91 Bulletin 91/04**

(51) Int. Cl.$^5$: **A61K 7/08**, A61K 7/50, C11D 1/12

(21) Application number: **86306934.0**

(22) Date of filing: **09.09.86**

(54) High oil containing anhydrous foamable compositions.

(30) Priority: **11.09.85 US 774728**

(43) Date of publication of application: **18.03.87 Bulletin 87/12**

(45) Publication of the grant of the patent: **23.01.91 Bulletin 91/04**

(84) Designated Contracting States: **AT BE CH DE FR GB IT LI LU NL SE**

(56) References cited:
EP-A- 0 203 750
FR-A- 2 097 333
FR-A- 2 214 746
FR-A- 2 274 684
GB-A- 460 839
GB-A- 2 150 436
US-A- 4 228 163

(73) Proprietor: CHESEBROUGH-POND'S INC.
Nyala Farm Road
Westport Connecticut 06881 (US)

(72) Inventor: Vishnupad, Mohan
79 Greenwood Lane
Monroe Connecticut 06468 (US)
Inventor: Ramirez, Jose E.
15 Fox Court
Trumbull Connecticut 06611 (US)
Inventor: Schmitt, William H.
103 Linden Avenue
Branford Connecticut 06405 (US)

(74) Representative: Froud, Clive et al
ELKINGTON AND FIFE Beacon House 113
Kingsway
London WC2B 6PP (GB)

## Description

This invention relates to high oil containing anhydrous foamable compositions. It is based on the discovery that petroleum jelly (also known as white petrolatum) unmodified or modified with mineral oil may be formulated with a certain organic detergent into anhydrous, cosmetically elegant creams which are unique in that they present adequate foaming properties, cleanse exceptionally well and simultaneously deposit a film of petrolatum on the surface. Because of the anhydrous cleansing compositions, many active drugs which in general are incompatible in presence of water may be incorporated into a medicated cleansing product.

More particularly, the present invention relates to essentially anhydrous base compositions containing an oily component of non-modified petroleum jelly or petroleum jelly modified with mineral oil and a mild detergent component, which is sodium cocoyl isethionate, in a high amount in order to impart to the base foaming characteristics when subsequently combined with water in use. The base compositions in accordance with the present invention are useful not only in cosmetic and pharmaceutical compositions, but also in cleansing compositions in general household use.

EP-A-203750 describes a synthetic detergent bar which may include an acyl isethionate as a surfactant and petrolatum and mineral oil as potential moisturizers. To achieve optimal foaming properties and skin feel, this reference requires a mixture of surfactants with the addition of up to 5% soap, and the use of polymeric skin feel and skin mildness aids. This contrasts with the present invention which requires merely a single mild detergent, sodium cocyl isethionate, to achieve good foaming and skin feel properties. Moreover, EP-A-203750 teaches the addition of 20-70% water to form a cream, thus preventing the resulting composition from being anhydrous like the present composition.

Prior to the present invention there have been many attempts to obtain foamable preparations containing significant amounts of an oily component such a petroleum jelly and mineral oil. Such preparations are generally emulsions containing substantial amounts of water in addition to a detergent component. These prior art emulsion systems have the drawbacks of not only foaming poorly, but also of becoming unstable thus rendering them non-functional and cosmetically unacceptable. Some of the difficulties encountered with such prior art compositions is that high concentrations of oleaginous hydrocarbons, such as petroleum jelly and mineral oil, generally tend to suppress the foaming properties of detergents thus reducing the basic function of the detergent.

Another drawback of the above-mentioned prior art compositions is that because of the presence of water in such compositions some therapeutic agents would be rendered prematurely active in such aqueous systems so that the therapeutic potency thereof would be lost during storage.

An object of the present invention is to provide high oil containing anhydrous compositions capable of foaming when combined with water during use.

Another object of the present invention is to provide high oil containing anhydrous compositions containing a mild and gentle detergent and capable of foaming when combined with water during use so as to provide useful cosmetic, pharmaceutical and cleansing compositions.

A further object of the present invention is to provide high oil containing anhydrous compositions capable of foaming when combined with water, but which provide a useful base for agents which are normally incompatible in water-containing foaming systems.

Yet another object of the present invention is to provide cosmetically elegant anhydrous foaming cream compositions.

A still further object of the present invention is to provide high oil containing anhydrous compositions which provide a stable and functional environment for therapeutic and/or cosmetic active ingredients which are incompatible in water-containing base systems.

It is also an object of the present invention to provide therapeutic compositions employing such anhydrous base compositions

It has been found that the objects of the present invention may be realized by combining with an oily component of petroleum jelly non-modified or modified with mineral oil, a detergent which is capable of wetting out easily in high concentrations with the oily component so as to form highly useful anhydrous compositions capable of foaming when combined with water during use.

Preferably the compositions in accordance with the present invention comprise the oily component in an amount of from 20 to 50%, by weight, preferably from 30 to 50%, by weight, of the total composition, active ingredients in an amount of from 0 to 20%, by weight, additives (e.g. humectants, inert fillers and colouring agents) in an amount of from 0 to 10%, by weight, and the remainder a mild detergent, sodium cocoyl isethionate.

In accordance with the present invention, high oil systems, such as petroleum jelly or petroleum jelly modified with mineral oil, for example, may be blended with a gentle and mild detergent sodium cocoyl isethionate to produce cosmetically useful anhydrous foaming creams. When the petroleum jelly is modified with mineral oil, the mineral oil may be in an amount of up to 20%, by weight, preferably up to 15%, by weight, of the total composition.

White petrolatum which is known as petroleum jelly is a purified mixture of semi-solid hydrocarbons obtained from petroleum, chiefly of the methane series of the general formula $CnH_{2n+}2$. The white to faintly yellowish unctuous mass has a density of 0.820-0.865, a melting point of 38-54°C and a refractive index of 1.460-1.474

In the present compositions the wetting properties of sodium cocoyl isethionate with the oily component is an important factor in the results obtained. Most detergents commonly used in cleansing products, when used in high concentrations of from 30 to 50% in the oil base, do not wet out easily, but form a hard lumpy mass which is cosmetically unacceptable and non-functional when combined with water. Generally, high oil containing compositions tend to suppress the foaming properties of detergents. While the present invention is not limited to any theory of action, it appears that in the present case the combination of high amounts of oil and high amounts of the mild detergent (sodium cocoyl isethionate) overcome the foam suppressing properties of the oil so as to provide an extremely functional, stable, good foaming and cosmetically attractive product.

A most important attribute of the anhydrous compositions in accordance with the present invention is to provide a vehicle for active ingredients which are incompatible in aqueous systems. The present anhydrous compositions not only provide desired cleansing and foaming activity, but also allow the incorporation of active ingredients which are incompatible with water-containing cleansing compositions. Examples of such active ingredients include :

1. Benzoyl peroxide for treating acne ;

2. Coal tar for treating psoriasis ; and,

3. The anti-microbial combination of the magnesium sulfate adduct of 2,2'-dithiobispyridine-1,1'-dioxide and a water-soluble zinc salt (e.g. zinc chloride) disclosed in EP-A-216558 (86 306 932.4).

The following method may be used to produce the composition in accordance with the present invention :

1. Pre-melt petroleum jelly in a jacketed kettle at 70°C.

2. Add sodium cocoyl isethionate to the batch and start homogenizing at 70°C.

3. Cool the batch to 55°C and optionally add fragrance while continuing mixing.

Optionally, if the composition contains pigments like titanium dioxide, humectants like glycerine and/or surfactant foam boosters like sodium lauryl sulfoacetate, each of these components are added to the batch in step 1 before step 2 is carried out. For example, the titanium dioxide may be premixed with glycerine and added to the batch of step 1. The sodium lauryl sulfoacetate is then added to the resulting mixture with continued mixing while maintaining the temperature at 70°C. Step 2 is then carried out.

The following are specific examples of compositions formed in accordance with the present invention.

Examples 1 - 3 relate to foamable skin cleansing compositions where the remaining Examples relate to medicinal skin treating compositions.

Example 1

```
Petroleum jelly              30.00

Mineral oil                  20.00

Sodium cocoyl isethionate    50.00
*Fragrance  and colour could be added as needed.
```

Example 2

```
Petroleum jelly              30.00
Mineral oil                  17.00
Glycerine                     5.00
Sodium cocoyl isethionate    47.00
Titanium dioxide              1.00
```

Example 3

```
Petroleum jelly              30.00
Mineral oil                  15.00
Glycerine                     5.00
Sodium cocoyl isethionate    44.25
Fragrance                     0.25
Sodium lauryl sulfoacetate    5.00
```

Example 4 (Medicated Acne Scrub)

```
Petroleum jelly              30.75
Mineral oil                  15.00
Benzoyl peroxide              5.00
Glycerine                     5.00
Sodium cocoyl isethionate    39.25
Sodium lauryl sulfoacetate    5.00
```

Example 5 (Antimicrobial Cleanser)

```
Petroleum jelly              30.00
Mineral oil                  15.00
Glycerine USP                 5.00
Titanium dioxide              0.50
Sodium cocoyl isethionate    40.50
Sodium lauryl sulfoacetate    5.00
Magnesium sulfate adduct of
   2,2'-dithio-bis-pyridine-
   1-oxide                    2.00
Zinc chloride (50% aqueous
   solution)                  2.00
```

4

Example 6 (Psoriatic Skin Cleanser)

| | |
|---|---|
| Petroleum jelly | 30.00 |
| Mineral oil | 10.00 |
| Coal tar solution | 5.00 |
| Glycerine | 5.00 |
| Titanium dioxide | 0.50 |
| Sodium cocoyl isethionate | 44.50 |
| Lathanol LAL | 5.00 |

## Claims

1. An anhydrous base composition characterised in that it comprises a high amount of an oily component of petroleum jelly non-modified or modified with mineral oil and sodium cocoyl isethionate in a high amount in order to impart thereto foaming characteristics when subsequently combined with water.

2. A composition as claimed in claim 1 wherein the oily component is in an amount of from about 20 to 50%, by weight, with the amount of mineral oil being in an amount of up to 20% by weight of the total composition, an active ingredient in an amount of from 0 to 20%, by weight, an additive in an amount of from 0 to 10%, by weight of the total composition and the remainder being sodium cocoyl isethionate.

3. A medicated acne skin composition characterised in that it comprises an anhydrous base composition as claimed in claim 1 or claim 2 and benzoyl peroxide in an amount conventionally used in topical compositions for the treatment of acne.

4. An anti-microbial cleanser composition characterised in that it comprises an anhydrous base composition as claimed in claim 1 or claim 2 and a combination of the magnesium sulfate adduct of 2,2'-dithiobis-pyridine-1,1'-dioxide and zinc chloride.

5. A psoriatic skin cleanser composition characterised in that it comprises an anhydrous base composition as claimed in claim 1 or claim 2 and coal tar.

## Ansprüche

1. Wasserfreie Grundmischung, dadurch gekennzeichnet, daß sie einen hohen Anteil einer öligen Komponente aus Petroljelly, welches nicht modifiziert ist oder welches mit Mineralöl und Natriumcocoylisethionat in hohem Anteil modifiziert ist, um dieser Schäumeigenschaften zu verleihen, wenn sie anschließend mit Wasser in Berührung kommt, enthält.

2. Mischung nach Anspruch 1, in welcher die ölige Komponente in einem Gewichtsanteil von 20-50 Gew.% enthalten ist, wobei der Anteil an Mineralöl bis zu 20 Gew.% bezogen auf die Gesamtzusammensetzung, ein Wirkstoff im Anteil von 0-20 Gew.%, ein Additiv im Anteil von 0-10 Gew.% der Gesamtmischung und der Rest Natriumcocoylisethionat sein kann.

3. Antiseptische Mischung zur Behandlung der Hautakne, dadurch gekennzeichnet, daß sie eine wasserfreie Grundmischung nach Anspruch 1 oder 2 und Benzoylperoxid in einem Anteil enthält, wie er üblicherweise in gebräuchlichen Mischungen zur Behandlung von Akne verwendet wird.

4. Antimikrobielle Reinigungszusammensetzung, dadurch gekennzeichnet, daß sie die wasserfreie Grundmischung nach Anspruch 1 oder 2 und eine Kombination aus dem Magnesiumsulfaltaddukt mit 2,2'-Dithio-bis-pyridin-1,1'-dioxid und Zinkchlorid besteht.

5. Mischung eines Reinigungsmittels für psoriatische Haut, dadurch gekennzeichnet, daß sie eine wasserfreie Grundmischung gem. Anspruch 1 oder Anspruch 2 und Steinkohlenteer enthält.

## Revendications

1. Une composition de base anhydre, caractérisée en ce qu'elle comprend une quantité élevée d'un composant huileux de vaseline non-modifiée ou modifiée par de l'huile minérale, et du cocoyl iséthionate de sodium dans une quantité élevée pour lui conférer des caractéristiques de moussage lorsqu'elle est ultérieurement combinée avec de l'eau.

2. Une composition comme revendiquée à la revendication 1, dans laquelle le composant huileux est présent dans une quantité allant d'environ 20 à 50%, en poids, la quantité d'huile minérale étant présente dans une quantité allant jusqu'à 20% en poids de la composition totale, un ingrédient actif, dans une quantité allant de 0 à 20% en poids, un additif, dans une quantité allant de 0 à 10%, en poids de la composition totale, et le reste étant du cocoyl iséthionate de sodium.

3. Une composition médicamenteuse pour peaux acnéiques, caractérisée en ce qu'elle comprend une composition de base anhydre comme revendiquée à la revendication 1 ou à la revendication 2 et du peroxyde de benzoyle dans une quantité utilisée de façon classique dans des compositions topiques pour le traitement de l'acné.

4. Une composition antimicrobienne de nettoyage, caractérisée en ce qu'elle comprend une composition de base anhydre comme revendiquée à la revendication 1 ou à la revendication 2 et une combinaison du produit d'addition du sulfate de magnésium sur le dithio-2,2' bis-pyridine-dioxyde-1,1' et du chlorure de zinc.

5. Une composition de nettoyage des peaux psoriasiques, caractérisée en ce qu'elle comprend une composition de base anhydre comme revendiquée à la revendication 1 ou à la revendication 2 et du goudron de houille.